# EUROPEAN PATENT APPLICATION

(11) **EP 4 155 419 A1**
(43) Date of publication of application: **29.03.2023**
(21) Application number: 21198612.0
(22) Date of filing: 23.09.2021
(51) Int. Cl.: C12Q 1/6841

(54) **MEANS AND METHOD FOR FLUORESCENT IN SITU HYBRIDIZATION USING CRISPR-CAS9 VARIANTS AND A NUCLEASE**

(71) Applicant: PixelBiotech GmbH, 69198 Schriesheim (DE)
(72) Inventor: Han, Kang, 69198 Schriesheim (DE); Liu, Sheng, 69198 Schriesheim (DE); Cheng, Yongsheng, 69198 Schriesheim (DE)
(74) Representative: Kuttenkeuler, David

(57) **Abstract**

The invention pertains to means and methods for fluorescent in situ hybridization (FISH) using a CRISPR CAS9 variant with nickase activity in order to denature the DNA doublestrand and then using an exonuclease in order to remove the DNA strand that is not targeted with the FISH probes. The invention enhances FISH signal to noise ratio and allows a highly specific targeting of genomic locations via the used sgRNA and FISH probe sequences. In addition, the invention provides uses and kits for the enhanced FISH methods.

## Description

### FIELD OF THE INVENTION

The invention pertains to means and methods for fluorescent in situ hybridization (FISH) using a CRISPR CAS9 variant with nickase activity in order to denature the DNA doublestrand and then using an exonuclease in order to remove the DNA strand that is not targeted with the FISH probes. The invention enhances FISH signal to noise ratio and allows a highly specific targeting of genomic locations via the used sgRNA and FISH probe sequences. In addition, the invention provides uses and kits for the enhanced FISH methods.

### DESCRIPTION

DNA fluorescence in *situ* hybridization (FISH) is a powerful technique in detecting chromosomal structures and has widely been used in the study of chromatin structure and clinical diagnosis of chromosomal abnormalities. However, DNA FISH needs genomic DNA to be opened first by heat denaturation and also requires a relatively long target region to generate a sufficient hybridization signal for detection. Usually, BAC (Bacterial Artificial Chromosome) based DNA FISH probes span dozens of kilobases (kb) or even hundreds of kb in genomic DNA. For fine chromatin structure studies, the heat denaturation and long target regions sometimes are prohibitive, especially when the target region is less than 10 kb long such as an exogenous gene that is introduced into the genome in cell and gene therapy.

The CRISPR-Cas9 (Streptococcus pyogenes) system has been extensively explored in studying genome structure both in *vivo* and *in vitro* (Chen et al., 2013; Deng et al., 2015; Ishii et al., 2019; Knight et al., 2015; Wang et al., 2021). The CRISPR-Cas9-sgRNA ribonucleoprotein complex can target specific genomic loci and label or open the genome for hybridization.

Fluorescent in situ hybridization (FISH) is currently used to identify specific DNA or RNA sequences in fixed cells and tissues. FISH typically uses a formamide chemistry and heat treatment for DNA denaturation. FISH includes steps for sample denaturation, hybridization, and post-hybridization washes, which require takes hours to days. (Lawrence JB, Villnave CA, Singer RH. Cell. 1988 Jan 15;52(1):51-61. PMID: 2830981 ; Lawrence JB, Singer RH. Nucleic Acids Res. 1985 Mar l l ; 13(5): 1777-99. PMID: 3889842; Cremer T. Landegent J. Brueckner A, Scholl HP, Schardin M, Hager HD, Devilee P. Pearson P. van der Ploeg M. (1986), "Detection of chromosome aberrations in the human interphase nucleus by visualization of specific target DNAs with radioactive and non-radioactive in situ hybridization techniques: diagnosis of trisomy 18 with probe L .84," Hum. Genet 74:346- 352; Pinkel D, Straume T. Gray JW. (1986) "Cytogenetic analysis using quantitative, high- sensitivity, fluorescence hybridization," Proc Natl Acad Sci USA 83 :2934-2938).

WO 2018/188856 discloses a novel FISH method for multiple labelling of nucleic acid probes by ligation. The method uses a ligase catalysed reaction to connect a nucleic acid probe with multiple pre-prepared nucleic acid label carrier molecules under the presence of a stabilizing complementary adaptor oligonucleotide. The method allows for an easy, cheap and fast labelling of multiple probes with multiple different labels. In this way, the costs and effort for the generation of single molecule Fluorescent In Situ Hybridization (smFISH) assays was significantly reduced, allowing combinatorial multi-color barcoding of nucleic acid probes The invention further provides methods for the generation of FISH libraries and labelling kits comprising the novel tools of the invention.

The international patent publication WO 2018/060249 on the other hand describes a multiple labelling FISH method that uses a ligase catalysed reaction to connect a nucleic acid probe with pre-prepared nucleic acid label carrier molecules under the presence of a stabilizing complementary splint oligonucleotide.

Despite continuous improvements, DNA FISH requires harsh treatments such as heat and formamide to denature dsDNA to allow probe hybridization, thus running the risk of affecting the integrity of the biological structure and genome organization. In cells, genomic DNA is highly folded and organized in three dimensions. The spatial organization of chromatin in the nucleus and the relative positions of distinct chromatin regions are tightly associated with gene regulation in normal development and disease. While it can be beneficial to study interphase genomic organization with DNA that is in its native state, the study of organization of chromatin is not possible with FISH methods that denature the native chromosomes.

### BRIEF DESCRIPTION OF THE INVENTION

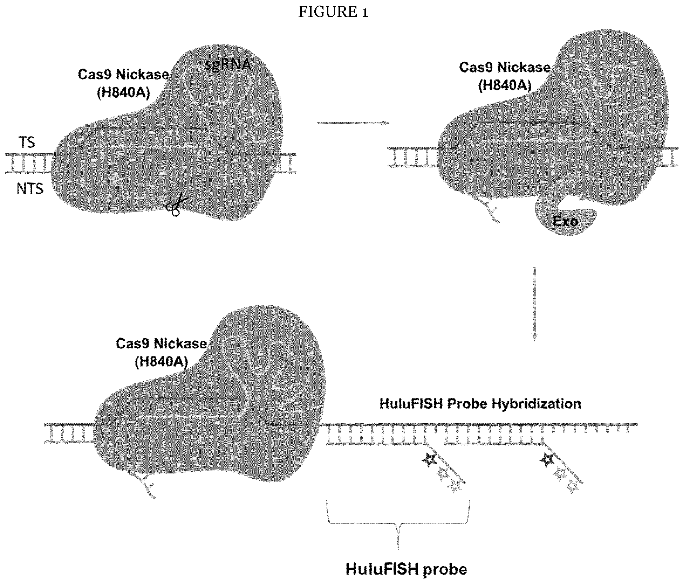

Here presented is a novel method called CRISPR-ExoFISH. The method is based on the CRISPR-Cas9 system to open specific genome loci and digest these loci to be single strand DNA (ssDNA), this providing permanent accessibility for DNA FISH probes (Figure 1). Once the DNA is opened by CRISPR-Cas9 (H840A mutant) on the target strand (TS), a T7 exonuclease starts to digest the non-targeting strand (NTS) of the CRISPR-Cas9 system and leaves the targeting strand intact for hybridization. For the following FISH probe hybridization, heat denaturation is not needed and a shorter region of less than 5kb is sufficient for the DNA FISH probe to bind and generate specific signals.

Generally, and by way of brief description, the main aspects of the present invention can be described as follows:
In **a first aspect,** the invention pertains to a method for fluorescent in situ hybridization (FISH), comprising the steps of
   - Targeted denaturing of a double stranded sample DNA using a DNA/RNA-guided DNA endonuclease as denaturing agent;
   - Hybridizing one or more FISH probes with the denatured sample DNA of (a);
   - Optionally, a washing step to remove unbound FISH probes; and
   - Detecting the binding of hybridized FISH probes.
In **a second aspect,** the invention pertains to a use of a DNA/RNA-guided DNA endonuclease, optionally together with an exonuclease, as denaturing agent in FISH.
In **a third aspect,** the invention pertains to a FISH kit, comprising one or more FISH probes, and at least a DNA/RNA-guided DNA endonuclease, optionally further comprising an exonuclease enzyme.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The presently-disclosed subject matter includes uses, kits, and methods for detecting nucleic acid sequences. Embodiments of the presently-disclosed subject matter can operate with or without requiring denaturation and/or fixation of a sample, and in some embodiments the probes can detect genomic DNA sequences. In this regard, because some embodiments of the presently-disclosed probes do not require denaturation of a sample, imaging methods that utilize the present probes can exclude time-consuming and sample- destroying denaturation steps that are commonly associated with other known imaging techniques. This, in turn, can enhance the rapidity with which the present probes can image nucleic acid sequences, which can be particularly advantageous for processes that are time sensitive, such as certain diagnostic, treatment, or screening processes.

In **the first aspect,** the invention pertains to a method for fluorescent in situ hybridization (FISH), comprising the steps of
- Targeted denaturing of a double stranded sample DNA using a DNA/RNA-guided DNA endonuclease as denaturing agent;
- Hybridizing one or more FISH probes with the denatured sample DNA of (a);
- Optionally, a washing step to remove unbound FISH probes; and
- Detecting the binding of hybridized FISH probes.

In the context of the present invention, the term "DNA/RNA guided DNA endonuclease" refers to DNA endonucleases that interact with at least one RNA/RNA guide molecule. Most preferably, the present invention will pertain to RNA guided DNA endonucleases. DNA endonucleases are enzymes that cleave the phosphodiester bond within a DNA polynucleotide chain. In case of RNA guided DNA endonuclease the interacting RNA-Molecule may guide the RNA guided DNA endonuclease to the site or location in a DNA where the endonuclease becomes active. In particular, the term RNA guided DNA endonuclease refers to naturally occurring or genetically modified Cas nuclease components or CRISPR-Cas systems, which include, without limitation, multi-subunit Cas protein effectors of class 1 CRISPR-Cas systems as well as single large effector Cas proteins of class 2 systems.

Details of the technical application of CRISPR/Cas systems and suitable RNA guided endonuclease are known to the skilled person and have been described in detail in the literature, as for example by Barrangou R et al. (Nat Biotechnol. 2016 Sep 8;34(9):933-941 ), Maeder ML et al. (Mol Ther. 2016 Mar;24(3):430-46) and Cebrian-Serrano A et al. (Mamm Genome. 2017; 28(7): 247-261 ). The present invention is not limited to the use of specific RNA guided endonucleases and therefore comprises the use of any given RNA guided endonucleases in the sense of the present invention suitable for use in the method described herein.

Any RNA guided DNA endonuclease known in the art may be employed in accordance with the present invention. RNA guided DNA endonuclease comprise, without limitation, Cas proteins of class 1 CRISPR-Cas systems, such as Cas3, Cas8a, Cas5, Cas8b, Cas8c, Cas10d, Cse1, Cse2, Csy1 , Csy2, Csy3, GSU0054, Cas10, Csm2, Cmr5, Csx1 1 , Csx10 and Csf1 ; Cas proteins of class 2 CRISPR-Cas systems, such as Cas9, Csn2, Cas4, Cpfl , C2c1 , C2c3 and C2c2; corresponding orthologous enzymes/CRISPR effectors from various bacterial and archeal species; engineered CRISPR effectors with for example novel PAM specificities, increased fidelity, such as SpCas9-HF1/eSpCas9, or altered functions, such as nickases, which are preferred in context of the present invention. Particularly preferred RNA guided DNA endonuclease of the present invention are Streptococcus pyogenes Cas9 (SpCas9), Staphylococcus aureus Cas9, Streptococcus thermophilus Cas9, Neisseria meningitidis Cas9 (NmCas9), Francisella novicida Cas9 (FnCas9), Campylobacter jejuni Cas9 (CjCas9), Cas12a (Cpf1) and Cas13a (C2C2) (Makarova KS et al. (November 2015). Nature Reviews Microbiology. 13 (11 ): 722-36).

Certain Cas enzymes suitable for the invention are Cas1B, Cas2, Cas3, Cas4, Cas5, Cash, Cas7, Cas8, Cas9, Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and Cpf1 polypeptide.

As such, the DNA/RNA-guided DNA endonuclease has preferably an activity to unwind double stranded sample DNA at least partially into two non-hybridized single DNA strands. Most preferred are genetically engineered Cas9 proteins, such as wherein the DNA/RNA-guided DNA endonuclease comprises an enzymatically non-functional or functionally altered nuclease domain. Most preferably, the DNA/RNA-guided DNA endonuclease is a Cas9 enzyme or a mutated Cas9 enzyme, such as preferably a Cas9 nickase such as a Cas9 comprising the H840A mutation.

The term "Cas9 nickase" refers to a modified version of the Cas9-gRNA complex, as described above, containing a single inactive catalytic domain, i.e., either the RuvC- or the HNH-domain. With only one active nuclease domain, the Cas9 nickase cuts only one strand of the target DNA, creating a single-strand break or "nick". A Cas9 nickase is still able to bind DNA based on gRNA specificity, though nickases will only cut one of the DNA strands. The majority of CRISPR plasmids currently being used are derived from S. pyrgenes and the RuvC domain can be inactivated by an amino acid substitution at position D10 (e.g., DIOA) and the HNH domain can be inactivated by an by an amino acid substitution at position H840 (e.g., H840A), or at positions corresponding to those amino acids in other proteins. As is known, the D10 and H840 variants of Cas9 cleave a Cas9-induced bubble at specific sites on opposite strands of the DNA. Depending on which mutant is used, the guide RNA-hybridized strand or the non-hybridized strand may be cleaved.

Also preferred in context of the invention are mutant Cas9 proteins that has inactivated nuclease activity. Such proteins refer to a Cas9 variant that has inactivated HNH and RuvC nucleases. Such a protein can bind to a target site in double-stranded DNA (where the target site is determined by the guide RNA), but the protein is unable to fully cleave the double-stranded DNA.

The core idea of the invention is an alternative method for denaturing double stranded DNA in order to render a sample accessible for FISH probes. Therefore, in context of the invention, the term "denaturing" or "denaturing DNA" refer to separating the two strands of a target double-strand DNA, including the complete removal of one of the strands, in context of the present invention, by the use of an exonuclease activity. Usual denaturing methods are therefore avoided, but in certain embodiments may still be used to further enhance the denaturing result of the invention. Denaturing DNA by any means known to a person skilled in the art includes, but is not limited to thorough pyrolysis (e.g., above 90° C.), alkali (e.g., NaOH) treatment, etc. The term "target DNA strand" as used in the present disclosure refers to the strand which the FISH probe used in the FISH method can be hybridized to in a subsequent step of the method of the invention.

The invention in particular uses a combination of a Cas9 nickase with an exonuclease in order to remove the "non-target" strand of the sample DNA which then renders the target region in a FISH experiment significantly more accessible to the FISH probes. Signal to noise ratios are significantly enhanced.

Therefore, in a preferred embodiment of the invention the herein disclosed methods do not involve the use of a helicase enzyme to unwind the double stranded sample DNA. The term "helicase" refers here to any enzyme capable of enzymatically unwinding a double stranded nucleic acid. For example, helicases are enzymes that are found in all organisms and in all processes that involve nucleic acid such as replication, recombination, repair, transcription, translation and RNA splicing. (Kornberg and Baker, DNA Replication, W. H. Freeman and Company (2nd ed. (1992)), especially chapter 11). Any helicase that translocates along DNA or RNA in a 5' to 3' direction or in the opposite 3' to 5' direction is excluded in present embodiments of the invention. This includes helicases obtained from prokaryotes, viruses, archaea, and eukaryotes or recombinant forms of naturally occurring enzymes as well as analogues or derivatives having the specified activity.

As mentioned, a preferred embodiment of the invention pertains to the use of a nickase in step (a) and wherein said step further comprises the use of an exonuclease, such as a bacteriophage exonuclease such as a T7- or Lambda exonuclease, and preferably wherein the exonuclease is suitable to remove the non-target DNA strand which is not targeted by the FISH probe. Generally, in context of the invention an exonuclease is a protein having exonuclease activity. As used herein, the term "exonuclease activity" is intended to mean hydrolysis of the phosphodiester bond that attaches a nucleotide to the end of a nucleic acid of length n to produce a nucleotide monophosphate and a nucleic acid of length n-1. The hydrolysis can occur at the bond that attaches the 5' nucleotide to the nucleic acid (i.e. 5' to 3' exonuclease activity) or at the bond that attaches the 3' nucleotide to the nucleic acid (i.e. 3' to 5' exonuclease activity). Exonucleases that are usable in context of the invention have an activity to remove the non-target strand about 20kB, preferably 10kB, upstream and/or downstream of a binding site of the DNA/RNA-guided DNA endonuclease. Further examples of exonucleases include, but are not limited to, lambda Exo, T7 Exo, Exol, Exo III, RecJf, Exo T BAL-31 nuclease and the like. A variety of exonucleases are known in the art and are available from commercial vendors

In certain preferred embodiments of the invention, the double stranded sample DNA is denatured by contacting the double stranded sample DNA with the DNA/RNA-guided DNA endonuclease and the exonuclease at the same time (concomitantly). In other embodiments, the double stranded sample DNA is contacted first with the DNA/RNA-guided DNA endonuclease (nickase) and subsequently with the exonuclease.

Certain prior art FISH methods apply the DNA binding activity of for example CRISPR proteins such as Cas9 as an alternative for purely nucleic acid based FISH probes. In such methods the DNA binding proteins are fluorescent labelled and used as probes for DNA detection. The present invention in certain preferred embodiments does not apply such protein-probes and hence, it is preferred that step (b) does not involve the use of DNA-binding protein linked FISH probes.

In any case, the present invention in certain embodiments may include that steps (a) and (b) are performed concomitantly.

In order to guide the DNA/RNA-guided DNA endonuclease to its target DNA, step (a) may comprise a contacting of the sample DNA with the DNA/RNA-guided DNA endonuclease and a guide RNA (gRNA) or guide DNA (gDNA), wherein the gRNA or gDNA have a sequence targeting one or more sequences known to be present in the sample DNA. It shall be understood, and as it is illustrated in for example figure 1, that the gRNA (or guide in genereal) is a nucleic acid molecule that has a sequence that is capable of complementary binding to the target DNA strand and not the non-target DNA strand. In certain preferred embodiments the FISH probe target sequence are in close proximity to each other, preferably not more than 20kb apart.

The term "gRNA" is used herein to refer to an RNA sequence selected to specifically target a particular nucleic acid sequence of interest (a "target nucleic acid sequence" or a "predetermined nucleic acid sequence"). Complexing between a Cas polypeptide and a gRNA can include the binding of the gRNA and polypeptide by covalent bonding, hydrogen bonding, and/or other non-eovalent bonding, and is well-understood in the field of CRISR/Cas systems. Exemplary gRNAs can therefore comprise a section that is targeted for a particular nucleic acid sequence of interest and section that is for binding to the Cas polypeptide, and these sections may or may not be mutually exclusive from one another. Moreover, additional sections may optionally be included in the gRNA.

In some embodiments the present invention includes a polypeptide that is conjugated to guide RNA (gRNA), wherein the gRNA is targeted to a predetermined nucleic acid sequence. In some embodiments, the gRNA has a target section. In some embodiments, the gRNA targets and hybridizes with the target nucleic acid sequence. The gRNA can comprise a single RNA molecule in some instances, and in other embodiments the gRNA can be assembled from two or more RNA molecules. The two or more RNA molecules individually may or may not be able to conjugate with the polypeptide, and may or may not target the predetermined nucleic acid sequences. However, upon assembly, the gRNA will perform to target a predetermined nucleic acid sequence.

The gRNA molecules utilized in embodiments of the present probes can vary in sequence length. The portion of the gRNA that has complementarity to a target nucleic acid sequence, the target section, can also vary in length, and can be selected so that the gRNA targets the target nucleic acid sequences (or "particular nucleic acid sequences of interest" or "predetermined nucleic acid sequences") of different lengths. In some embodiments the portion of the gRNA targeted to a predetermined nucleic acid sequence, the predetermined nucleic acid sequence, or the entire gRNA sequence is about 1, 2, 3, 4, 5, 6, 7, 8, 9, 10, 11, 12, 13, 14, 15, 16, 17, 18, 19, 20, 21 , 22, 23, 24, 25, 26, 27, 28, 29, 30 ,31 ,32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more nucleic acids in length. In some preferred embodiments, the target section of the gRNA is about 15-25 nucleic acids in length. In some embodiments, the gRNA has a section binding to the polypeptide but not the target nucleic acid. In some embodiments, this section is about 80 to about 100 nucleic acids in length, more preferably about 90 nucleic acids in length.

Methods and tools for designing gRNAs are known to those of ordinary skill in the art. For example, there are multiple publicly-available tools that can be accessed at the following sites: crispr.mit.edu/; https://chopchop.rc.fas.harvard.edu/; www.e-crisp.org/; crispr. cos. un i-heidelberg. de/ [0098] The terms "target," "targeting," and the like refer to the characteristic of a compound being selectively drawn to and/or selectively bound a target compound. For instance, gRNA that targets a predetermined DNA sequence is drawn to and/or selectively binds the predetermined DNA sequence. In some embodiments a gRNA molecule is said to target a predetermined DNA sequence when the gRNA is hybridizable with the predetermined DNA sequence, wherein the term "hybridize" refers to the binding (e.g., non- covalent binding) of one nucleic acid sequence to another nucleic acid sequence in a sequence specific manner. Hybridizable sequences can therefore be referred to as "complementary" sequences herein.

In some instances 100% complementarity of the target section of a nucleic acid (e.g., gRNA) to its target nucleic acid (e.g., DNA) is not required for hybridization. In some instances, a nucleotide can hybridize over one or more segments such that intervening or adjacent segments are not involved in the hybridization event (e.g., a loop structure or hairpin structure). The target section of a nucleotide can comprise at least 50%, at least 60%, at least 70%, at least 80%, at least 90%, at least 95%, at least 99%, or 100% sequence complementarity to a hybridizable target sequence.

"Complementarity" refers to the ability of a nucleic acid to form hydrogen bond(s) with another nucleic acid sequence by either traditional Watson-Crick or other non- traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 50%, 55%, 60%, 65%, 70%, 75%, 80%, 85%, 90%, 95%. 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part 1, Second Chapter "Overview of principles of hybridization and the strategy of nucleic acid probe assay", Elsevier, N.Y.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

The terms "nucleotide," "polynucleotide," "nucleic acid," and "nucleic acid sequence" are also used herein to refer to deoxyribonucleotides or ribonucleotides and polymers thereof in either single or double stranded form. Unless specifically limited, the terms encompass nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified versions thereof (e.g., degenerate codon substitutions) and complementary sequences and as well as the sequence explicitly indicated. Thus, the term nucleotide and the like is inclusive of the gRNAs that are described herein.

The terms "predetermined nucleic acid sequence," "target nucleic acid sequence," and "particular nucleic acid sequence of interest" refer to a nucleic acid sequence of a nucleic acid molecule that is known and was chosen before synthesis of the gRNA in accordance with the invention disclosed herein. Accordingly, in its broadest sense the expression "target nucleic acid sequence" indicates merely that target nucleic acid sequence has a particular known sequence.

The target nucleic acid sequence can be selected from a DNA sequence, an RNA sequence, or a sequence of another nucleic acid analogue such as but not limited to peptide nucleic acid (PNA), Morpholino and locked nucleic acid (LNA), as well as glycol nucleic acid (GNA) and threose nucleic acid (TNA). In some embodiments, the target nucleic acid sequence is a nucleic acid molecule in which one or more nucleotides have been modified such as but not limited to methylation, and hydroxymethylation. As noted herein, the target nucleic acid sequence can be in native DNA that has not been denatured, and the target nucleic acid sequence can be within chromosomal DNA that has not been denatured.

The target nucleic acid sequence can be contained in any sample containing genetic material. In this regard, the probes, kits, and methods disclosed herein are useful for detecting and imaging target nucleic acid sequence(s) in a sample(s) containing genetic material.

The sample types are not particularly limited so long as the samples comprise genetic material. In some embodiments, the cell is a human cell. In some embodiments, the sample comprises a virus, bacteria or fungus. In some embodiments, the sample is a cell. The cell can be a live cell or a fixed cell. The cell can be a plant cell or an animal cell. In some embodiments the sample includes an entire native chromosome and/or a portion of a native chromosome. In some embodiments, the cell can be a human cell.

Thus, in some embodiments the samples include cells obtained from a subject. Exemplary samples from a subject may include chromosome spreads for genetic testing, cultures, prenatal materials, samples for in vitro fertilization, swabs, air filters, water cooling towers, food, drink, hair, stool, urine, saliva, blood, lymph, sputum, cervical smears, sperm, sections of tissues from biopsies, sections of tissues from autopsies, and the like. Samples can include fixed tissue samples by various fixation methods known in the art. Examples of chemical fixation include formaldehyde, paraformaldehyde, formamide, glutaraldehyde, Tween-20 and HC1, acetic acid and methanol, and other aldehydes and alcohols.

In one preferred embodiment of the invention the sgRNA or (gRNA) target sequence is a non-redundant sequence and binding specifically in proximity to the suspected FISH probe target sequence, whereby signal to noise ratio of the in situ hybridization is increased. This embodiment relates to a variation of the method wherein the binding sites for the FISH probe and the guide DNA/RNA is carefully designed to exclude unspecific signals. If the FISH experiments is used to detect the presence or absence of sequences in a very specific genomic location, one may design guide sequences to be so specific that they will bind a site in close proximity to the site where the FISH probe is suspected to hybridize. Close proximity shall reflect a genomic distance that in which the present invention can denaturate the target double strand, therefore depends on the exonuclease enzyme and how of the non-target strand can be removed. Hence, the sgRNA or sgDNA may hybridize to (and direct the Cas9 to) the FISH target strand, which is the strand in the double stranded sample DNA which is suspected to hybridize to the FISH probes.

As an example of such a method the sgRNA or sgDNA have a targeting sequence which targets a repetitive sequence in the sample DNA or which targets a non-repetitive sequence in the sample DNA.

The advantage of the present method is that no other, usually harsh denaturing procedures are required. Therefore, in certain preferred embodiments no heat-assisted denaturing is used. More preferably no other denaturing procedure is used during the method of the invention.

The invention is in particular useful for an application of multiple labelled FISH probes. Hence, a FISH probe in context of the present invention is preferably a FISH probe having two or more different fluorescent labels. In any case, a FISH probe is preferably a probe as produced according to a method disclosed in one of the international applications published as WO 2018/188856 and/or WO 2018/060249 (each of which are hereby incorporated by reference in their entirety). In certain preferred embodiments, a FISH probe used in context of the invention is produced by multiple way ligation as described in WO 2018/188856. Such procedure involves the use of a probe sequence oligonucleotide, a label carrier oligonucleotide and an adaptor oligonucleotide (which each are as defined in WO 2018/188856). However, the adaptor may be removed before use.

In **the second aspect,** the invention pertains to a use of a DNA/RNA-guided DNA endonuclease, optionally together with an exonuclease, as denaturing agent in FISH. A use in accordance to the invention is preferred wherein the DNA/RNA-guided DNA endonuclease has an activity to unwind double stranded sample DNA at least partially into two non-hybridized single DNA strands. As another embodiment, the DNA/RNA-guided DNA endonuclease comprises an enzymatically non-functional or functionally altered nuclease domain.

In certain preferred embodiments, the invention provides such a use wherein the DNA/RNA-guided DNA endonuclease is a Cas9 enzyme or a mutated Cas9 enzyme, such as preferably a Cas9-nickase such as a Cas9 comprising the H840A mutation.

Most preferably the use of the invention is a use in a method of the first aspect.

In **the third aspect,** the invention pertains to a FISH kit, comprising one or more FISH probes, and at least a DNA/RNA-guided DNA endonuclease, optionally further comprising an exonuclease enzyme.

A preferred kit is further comprising an exonuclease, such as a bacteriophage exonuclease such as a T7- or Lambda-exonuclease.

Another preferred kit is a kit of the invention, wherein the DNA/RNA-guided DNA endonuclease has an activity to unwind double stranded sample DNA at least partially into two non-hybridized single DNA strands.

Another preferred kit is a kit of the invention, wherein the DNA/RNA-guided DNA endonuclease comprises an enzymatically non-functional or functionally altered nuclease domain.

Another preferred kit is a kit of the invention, wherein the DNA/RNA-guided DNA endonuclease is a Cas9 enzyme or a mutated Cas9 enzyme, such as preferably a Cas9-nickase such as a Cas9 comprising the H840A mutation.

Another preferred kit is a kit of the invention, further comprising an exonuclease enzyme.

Another preferred kit is a kit of the invention which does not comprise a helicase enzyme.

A kit of the invention may further comprise any components of a kit as disclosed in either WO 2018/188856 and/or WO 2018/060249. Such a kit is suitable in order to conduct a FISH experiment as disclosed in that documents, but with a denaturing method described herein.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1** is a depiction of the CRISPR-ExoFISH workflow. Genomic DNA loci are bound by Cas9 nickase (H840A)-sgRNA ribonucleoprotein complex and cut at the NTS strand. Exonuclease finds the flapped DNA on the NTS strand and digests the NTS stand. The TS strand is then used as a target for hybridization by HuluFISH probes (labeling indicated by stars).

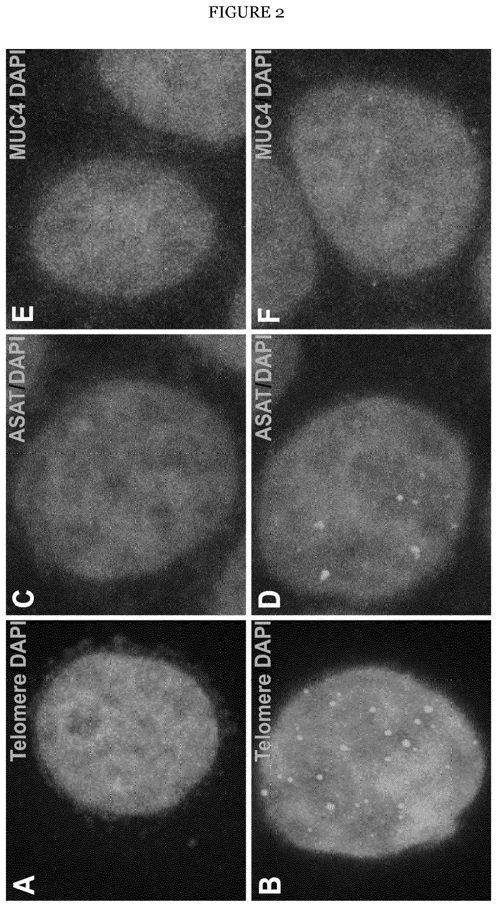

**Figure 2** shows CRISPR-ExoFISH on Human genomic targets. (A, C, E) Hela cell stained with HuluFISH probe for telomere, ASAT, and MUC4 respectively. (B, D, F) Hela cell were fixed and stained with HuluFISH probe against Human telomere, ASAT, and MUC4 respectively, after genomic DNA of telomere, ASAT and MUC4 is opened and digested as ssDNA.

The sequences show:

| **SEQ ID NO:** | **Sequence:** | **Name:** | **Note:** |
|---|---|---|---|
| 1 | TTCTAATACGACTCACTATAGGGATGAATA taagggtaagggtaagggtaaGTTTTAGAG | Telomere_gRNA | Oligo DNA template for sgRNA in Telomere |
| | CTAGA | | |
| 2 | TTCTAATACGACTCACTATAGGGATGAATA ctttttgtagaatctgcaagGTTTTAGAGC TAGA | ASAT_gRNA | Oligo DNA template for sgRNA in alpha satellite |
| 3 | TTCTAATACGACTCACTATAGGGATGAATA gcgatgtctggaaggatccaGTTTTAGAGC TAGA | MUC4NR_gRNA_7 | Oligo DNA template for sgRNA in MUC4 intron 1 |
| 4 | TTCTAATACGACTCACTATAGGGATGAATA ggtgagacgcagacaaactgGTTTTAGAGC TAGA | MUC4NR_gRNA_210 | Oligo DNA template for sgRNA in MUC4 intron 1 |
| 5 | TTCTAATACGACTCACTATAGGGATGAATA ttgggtcacggccttgcctgGTTTTAGAGC TAGA | MUC4NR_gRNA_410 | Oligo DNA template for sgRNA in MUC4 intron 1 |
| 6 | TTCTAATACGACTCACTATAGGGATGAATA ctggggtccagagttcaagcGTTTTAGAGC TAGA | MUC4NR_gRNA_620 | Oligo DNA template for sgRNA in MUC4 intron 1 |
| 7 | TTCTAATACGACTCACTATAGGGATGAATA caggactaggctgagggcaaGTTTTAGAGC TAGA | MUC4NR_gRNA_821 | Oligo DNA template for sgRNA in MUC4 intron 1 |
| 8 | TTCTAATACGACTCACTATAGGGATGAATA gaggctggggcttggggcgcGTTTTAGAGC TAGA | MUC4NR_gRNA_1025 | Oligo DNA template for sgRNA in MUC4 intron 1 |
| 9 | TTCTAATACGACTCACTATAGGGATGAATA aagctgagcccaaatgtgctGTTTTAGAGC TAGA | MUC4NR_gRNA_1225 | Oligo DNA template for sgRNA in MUC4 intron 1 |
| 10 | TTCTAATACGACTCACTATAGGGATGAATA ctgagtcctttgcgtcgctaGTTTTAGAGC TAGA | MUC4NR_gRNA_1434 | Oligo DNA template for sgRNA in MUC4 intron 1 |
| 11 | TTCTAATACGACTCACTATAGGGATGAATA acctgcctatacttgagtctGTTTTAGAGC TAGA | MUC4NR_gRNA_1662 | Oligo DNA template for sgRNA in MUC4 intron 1 |
| 12 | TTCTAATACGACTCACTATAGGGATGAATA gatggaaatttggtgtctacGTTTTAGAGC TAGA | MUC4NR_gRNA_1870 | Oligo DNA template for sgRNA in MUC4 intron 1 |
| 13 | TTAGGGTTAGGGTTAGGGTTAGGGTTAGGG | HuluFISH_Telomere | HuluFISH probe sequence for Telomere |
| 14 | CTCACAGAGTTGAACCTTCCT | ASAT_GSO_AS | HuluFISH probe sequence for alpha satellite DNA |
| 15 | CCCAGGCTTACTCGCAGA | MUC4_NR_52 | HuluFISH probe MUC4 intron 1 |
| 16 | GAGAAAGACAGGCAGAGC | MUC4_NR_70 | HuluFISH probe MUC4 intron 1 |
| 17 | CAGAGCAAGAGGAACAGAGTC | MUC4_NR_91 | HuluFISH probe MUC4 intron 1 |
| 18 | AAGGAGAAAGATGTACACCC | MUC4_NR_111 | HuluFISH probe MUC4 intron 1 |
| 19 | TTGTGTACAGAGCTGGGGG | MUC4_NR_130 | HuluFISH probe MUC4 intron 1 |
| 20 | TAGAGGGGATGCCAGGAAAG | MUC4_NR_150 | HuluFISH probe MUC4 intron 1 |
| 21 | CTGGGTGATGGAGACGGAAG | MUC4_NR_170 | HuluFISH probe MUC4 intron 1 |
| 22 | AACTCATGTAAAGCTGCAGGG | MUC4_NR_193 | HuluFISH probe MUC4 intron 1 |
| 23 | AGGACCCTGGGAATGGAGA | MUC4_NR_247 | HuluFISH probe MUC4 intron 1 |
| 24 | GAGGAGAAGATCGGGAG | MUC4_NR_264 | HuluFISH probe MUC4 intron 1 |
| 25 | CAGCAAGCAAGGGAAGC | MUC4_NR_285 | HuluFISH probe MUC4 intron 1 |
| 26 | ACAAGGAGGAGAGGGGCA | MUC4_NR_304 | HuluFISH probe MUC4 intron 1 |
| 27 | CCGTGGGCTTCTTACCTCTG | MUC4_NR_385 | HuluFISH probe MUC4 intron 1 |
| 28 | AGCTCGGGTTTAAAAGC | MUC4_NR_402 | HuluFISH probe MUC4 intron 1 |
| 29 | CCTTGGGCTCCTCCGTGTACA | MUC4_NR_471 | HuluFISH probe MUC4 intron 1 |
| 30 | CCAGGCCTGTGGGAGATGTT | MUC4_NR_511 | HuluFISH probe MUC4 intron 1 |
| 31 | CCCGTCCTCTTCCCCACACTT | MUC4_NR_544 | HuluFISH probe MUC4 intron 1 |
| 32 | GCTGTCCCTCTGGCTTCA | MUC4_NR_567 | HuluFISH probe MUC4 intron 1 |
| 33 | ATTCTGTGTTTCTGGGTGTC | MUC4_NR_599 | HuluFISH probe MUC4 intron 1 |
| 34 | TGAGTCTTTGGGGGAGAGT | MUC4_NR_618 | HuluFISH probe MUC4 intron 1 |
| 35 | TGGGGTTAGAGTCTCAGCTCC | MUC4_NR_659 | HuluFISH probe MUC4 intron 1 |
| 36 | CCTCTCAGCAGGCTAAGAACA | MUC4_NR_687 | HuluFISH probe MUC4 intron 1 |
| 37 | GTCGCCGAGGGAAAATATTTC | MUC4_NR_708 | HuluFISH probe MUC4 intron 1 |
| 38 | TTGGGCGCATATTTGAGGAG | MUC4_NR_728 | HuluFISH probe MUC4 intron 1 |
| 39 | CTTCCTGGGAGTGAGTCAGA | MUC4_NR_748 | HuluFISH probe MUC4 intron 1 |
| 40 | CGAGTGCCGTTTAAAGG | MUC4_NR_768 | HuluFISH probe MUC4 intron 1 |
| 41 | CTGCAAGAGAAGCCATGCTG | MUC4_NR_788 | HuluFISH probe MUC4 intron 1 |
| 42 | CCTTCCACCTCGGGATGTT | MUC4_NR_818 | HuluFISH probe MUC4 intron 1 |
| 43 | GCTCCAGTGCTCATCCCA | MUC4_NR_929 | HuluFISH probe MUC4 intron 1 |
| 44 | TGCCCTGCAGATGTGGTTG | MUC4_NR_989 | HuluFISH probe MUC4 intron 1 |
| 45 | GGAGTCTCCACGGGAATCA | MUC4_NR_1010 | HuluFISH probe MUC4 intron 1 |
| 46 | CGGGGAGGCAGCTTGGATTT | MUC4_NR_1063 | HuluFISH probe MUC4 intron 1 |
| 47 | CCCCAGGGCCAAGTCTTT | MUC4_NR_1086 | HuluFISH probe MUC4 intron 1 |
| 48 | GCCGTGAACTGTTCTGG | MUC4_NR_1103 | HuluFISH probe MUC4 intron 1 |
| 49 | GCCCCGGTCCCCTGTGTAAAA | MUC4_NR_1160 | HuluFISH probe MUC4 intron 1 |
| 50 | AGCAGTGGTGAACGGTTGG | MUC4_NR_1179 | HuluFISH probe MUC4 intron 1 |
| 51 | ACCTCCTGACGCCCAAGTT | MUC4_NR_1198 | HuluFISH probe MUC4 intron 1 |
| 52 | CTGGACACTCAGCTCCATGT | MUC4_NR_1272 | HuluFISH probe MUC4 intron 1 |
| 53 | CAGGAAGTGGGGTCTGTGGA | MUC4_NR_1297 | HuluFISH probe MUC4 intron 1 |
| 54 | ACCTGGGGTGGGCGTGGAAAA | MUC4_NR_1338 | HuluFISH probe MUC4 intron 1 |
| 55 | CGTGACCCGGAGAAGGAGTGA | MUC4_NR_1368 | HuluFISH probe MUC4 intron 1 |
| 56 | AGGACTGTTGGTGTGCAAGG | MUC4_NR_1388 | HuluFISH probe MUC4 intron 1 |
| 57 | GTCTCCATGACGACCCGAA | MUC4_NR_1409 | HuluFISH probe MUC4 intron 1 |
| 58 | GAAGCTAGGCATGTCGTGGA | MUC4_NR_1429 | HuluFISH probe MUC4 intron 1 |
| 59 | AAGTGGAGCTGGGCCAGGAGA | MUC4_NR_1481 | HuluFISH probe MUC4 intron 1 |
| 60 | GAGATGGTCGTGGCTGGGAGA | MUC4_NR_1503 | HuluFISH probe MUC4 intron 1 |
| 61 | TGGCACCCACACATCTGAC | MUC4_NR_1522 | HuluFISH probe MUC4 intron 1 |
| 62 | CTTGGCAGGAAAAGCAGTCAC | MUC4_NR_1559 | HuluFISH probe MUC4 intron 1 |
| 63 | CCAGCCAGCCCCGTTCCTTTT | MUC4_NR_1635 | HuluFISH probe MUC4 intron 1 |
| 64 | CCTGAAGGGGTTTACAGATG | MUC4_NR_1660 | HuluFISH probe MUC4 intron 1 |
| 65 | AGGGTCTGTTTGCACACTTG | MUC4_NR_1700 | HuluFISH probe MUC4 intron 1 |
| 66 | CCAGGCTGGGTCACACTGAA | MUC4_NR_1736 | HuluFISH probe MUC4 intron 1 |
| 67 | GCCCAGGCCAGAGGAAAAA | MUC4_NR_1755 | HuluFISH probe MUC4 intron 1 |
| 68 | CACAGGGTTTCCACAAAG | MUC4_NR_1773 | HuluFISH probe MUC4 intron 1 |
| 69 | GCTGCCGCAATGAGGGTTT | MUC4_NR_1794 | HuluFISH probe MUC4 intron 1 |
| 70 | CCTTAAGGAACAGCCCTGGC | MUC4_NR_1814 | HuluFISH probe MUC4 intron 1 |
| 71 | CAAGGGTTAAAGGATAAGGC | MUC4_NR_1837 | HuluFISH probe MUC4 intron 1 |
| 72 | CAGCAGACAGAGGTGGGCTA | MUC4_NR_1858 | HuluFISH probe MUC4 intron 1 |
| 73 | CAGGCAGGAATGACTCAGA | MUC4_NR_1916 | HuluFISH probe MUC4 intron 1 |
| 74 | CCGTCCTGGTTCCATGCCACA | MUC4_NR_1948 | HuluFISH probe MUC4 intron 1 |
| 75 | GGCAACTGGGTCGAAATAGG | MUC4_NR_1974 | HuluFISH probe MUC4 intron 1 |
| 76 | CCTTGGTCTCCAGCACTATCA | MUC4_NR_1995 | HuluFISH probe MUC4 intron 1 |

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: CRISPR-ExoFISH is used to specifically stain three genomic targets in Hela cells.

### Methods and Materials

**sgRNA synthesis by *in vitro* transcription (IVT):** sgRNA was produced using a ENGen SgRNA synthesis kit (NEB, E3322). sgRNA guide sequences were designed by customized script at PixelBiotech GmbH for Human Telomere, alpha-satellite DNA (ASAT), and the non-repetitive region of MUC4 gene (Intron 1, 2kb long target). Target-specific DNA oligo templates were designed according to the protocol of the ENGen SgRNA synthesis kit. The sgRNA IVT was following the protocol of the kit. For multiple sgRNA synthesis in one reaction, the total concentration of oligo DNA template was adjusted to 250 nM final concentration. The sgRNA was purified using a Monarch RNA Cleanup kit (NEB, T2040).

**CRISPR-ExoFISH:** Hela cells were fixed by cold methanol:acetic (3:1, stored at ― 20 °C) for 10 min and stored at ―20 °C or immediately used. RNase A (NEB, T3018) treatment at 0.1mg/ml in the 1×PBS for 30 min at 37°C is optional to remove background staining from cellular RNA. After RNase A treatment, the cells were washed with 1×PBS at 65 °C for 10 min each, 3 times. Cas9-sgRNA ribonucleoprotein complex was reconstituted in binding buffer (20 mM HEPES, pH 7.5, 100mM KCl, 5 mM MgCl₂, 5% (v/v) Glycerol, 0.1% (v/v) Tween-20, 1% (w/v) Bovine serum albumin (BSA), 1 mM Dithiothreitol (DTT), 0.4 U/µl RNase inhibitor Murine (NEB, M0314)) at the concentration of 200 nM each (1:1 ratio for Cas9:sgRNA concentration) for 10 min at room temperature. Fixed cells were washed with the binding buffer for 10 min at room temperature. Then the cells were incubated with 200 nM Cas9-sgRNA ribonucleoprotein complex in the binding buffer with 1 U/ul T7 exonuclease (NEB, M0263) for 1 hour at 37 °C. Then the cells were washed with 1×PBS for 5 min. After washing with 1×PBS for 3 times in 5 min, cells were hybridized with a 320 nM HuluFISH probe in HuluHyb (PixelBiotech GmbH, PB102) for 1 hours at 37 °C. After hybridization, the cells were washed with 2×SSC, 20% formamide at 37 °C for 10 min for two times, and then washed with 1×PBS for 5 min before mounting in the VectorShield HardSet (Vector Laboratories, H-1400). Stained cells were imaged under Leica TCS SP8 confocal microscope.

### Results

CRISPR-ExoFISH was tested on 3 targets in human cells. In Figure 2A-B, telomere sgRNA was designed against the repeat TAAGGG in Human telomeres (sequence details in table 1). Without sgRNA and Cas9, HuluFISH probes for telomeres could not bind at all to the genome without heat denaturation. In case of facilitation by the Cas9-sgRNA complex, HuluFISH probes efficiently hybridize to the telomere region. This indicates the ssDNA nature of the telomeres after Cas9-sgRNA binding and T7 exonuclease digestion. For further validation of this novel method, another repeat region in the genome, alpha satellite DNA (ASAT) was chosen (Figure 2C-D). All repeated genomic regions show specific staining compared to the negative control without Cas9-sgRNA opening and digesting the genomic DNA.

Despite the repeat region, CRISPR-ExoFISH was also employed to detect non-repetitive genomic loci. MUC4 intron 1 was chosen for validation (Figure 2E-F). A 2kb region was selected for sgRNA design and 9 sgRNAs were selected for in vitro transcription and assembly with Cas9. Additionally 63 HuluFISH probes were designed for the 2kb region in MUC4. The results showed specific staining of the MUC4 gene intron 1.

### REFERENCES

The references are:
Chen, B., Gilbert, L.A., Cimini, B.A., Schnitzbauer, J., Zhang, W., Li, G.-W., Park, J., Blackburn, E.H., Weissman, J.S., Qi, L.S., et al. (2013). Dynamic Imaging of Genomic Loci in Living Human Cells by an Optimized CRISPR/Cas System. Cell 155, 1479―1491.
Deng, W., Shi, X., Tjian, R., Lionnet, T., and Singer, R.H. (2015). CASFISH: CRISPR/Cas9-mediated in situ labeling of genomic loci in fixed cells. Proc. Natl. Acad. Sci. 112, 11870-11875.
Ishii, T., Schubert, V., Khosravi, S., Dreissig, S., Metje - Sprink, J., Sprink, T., Fuchs, J., Meister, A., and Houben, A. (2019). RNA - guided endonuclease - in situ labelling (RGEN - ISL): a fast CRISPR/Cas9 - based method to label genomic sequences in various species. New Phytol. 222, 1652―1661.
Knight, S.C., Xie, L., Deng, W., Guglielmi, B., Witkowsky, L.B., Bosanac, L., Zhang, E.T., Beheiry, M.E., Masson, J.-B., Dahan, M., et al. (2015). Dynamics of CRISPR-Cas9 genome interrogation in living cells. Science 350, 823―826.
Wang, Y., Cottle, W.T., Wang, H., Feng, X.A., Mallon, J., Gavrilov, M., Bailey, S., and Ha, T. (2021). Genome oligopaint via local denaturation fluorescence in situ hybridization. Mol. Cell 81, 1566-1577.e8.

## Claims

1. **A method for fluorescent in situ hybridization (FISH),** comprising the steps of
(a) Targeted denaturing of a double stranded sample DNA using a DNA/RNA-guided DNA endonuclease and an exonuclease as denaturing agents;
(b) Hybridizing one or more FISH probes with the denatured sample DNA of (a);
(c) Optionally, a washing step to remove unbound FISH probes; and
(d) Detecting the binding of hybridized FISH probes.

2. The method of claim 1, wherein the method does not involve the use of a helicase enzyme to unwind the double stranded sample DNA during step (a), preferably the method does not comprise the use of a helicase.

3. The method of any one of claims 1 to 2, wherein the DNA/RNA-guided DNA endonuclease is a Cas9 enzyme or a mutated Cas9 enzyme, such as preferably a Cas9 nickase such as a Cas9 comprising the H840A mutation (CAS9 nickase).

4. The method of claim 3, wherein the exonuclease is a bacteriophage exonuclease such as a T7- or Lambda exonuclease, and preferably wherein the exonuclease is suitable to remove the non-target DNA strand which is not targeted by the FISH probe.

5. The method of claim 4, wherein the double stranded sample DNA is denatured by contacting the double stranded sample DNA with the DNA/RNA-guided DNA endonuclease and the exonuclease at the same time (concomitantly).

6. The method of any one of claims 1 to 5, wherein steps (a) and (b) are performed concomitantly.

7. The method of any one of claims 1 to 6, wherein step (a) comprises a contacting of the sample DNA with the DNA/RNA-guided DNA endonuclease and a guide RNA (sgRNA) or guide DNA (sgDNA), wherein the sgRNA or sgDNA have a sequence targeting one or more sequences known to be present in the sample DNA.

8. The method of any one of claims 1 to 7, wherein no heat-assisted denaturing is used.

9. The method of any one of claims 1 to 16, wherein the FISH is a FISH method as disclosed in WO 2018/188856 and/or WO 2018/060249.

10. **A use of a DNA/RNA-guided DNA endonuclease,** together with an exonuclease, as denaturing agent in FISH.

11. The use of claim 10, wherein the DNA/RNA-guided DNA endonuclease is a Cas9 enzyme or a mutated Cas9 enzyme, such as preferably a Cas9-nickase such as a Cas9 comprising the H840A mutation.

12. **A FISH kit,** comprising one or more FISH probes, and at least a DNA/RNA-guided DNA endonuclease and an exonuclease.

13. The FISH kit of claim 12, wherein the exonuclease is a bacteriophage exonuclease such as a T7- or Lambda-exonuclease.

14. The FISH kit of claim 12 or 13, wherein the DNA/RNA-guided DNA endonuclease is a Cas9 enzyme or a mutated Cas9 enzyme, such as preferably a Cas9-nickase such as a Cas9 comprising the H840A mutation.

15. The FISH kit of any one of claims 12 to 14 which does not comprise a helicase enzyme.
